(19) Europäisches Patentamt
European Patent Office
Office européen des brevets

(11) Veröffentlichungsnummer: **0 165 521**
**B1**

# (12) EUROPÄISCHE PATENTSCHRIFT

(45) Veröffentlichungstag der Patentschrift: **22.04.87**

(21) Anmeldenummer: **85106763.7**

(22) Anmeldetag: **01.06.85**

(51) Int. Cl.[4]: **C 07 C 67/343**, C 07 C 69/618, C 07 C 69/734, C 07 C 57/44, C 07 C 27/02

## (54) Verfahren zur Herstellung eines Gemisches aus einem gegebenenfalls substituierten Zimtsäureester und einem gegebenenfalls substituierten Beta-Alkoxy-Beta-phenyl-propionsäureester sowie Verfahren zur Herstellung von gegebenenfalls substituierter Zimtsäure.

(30) Priorität: **16.06.84 DE 3422412**

(43) Veröffentlichungstag der Anmeldung: **27.12.85 Patentblatt 85/52**

(45) Bekanntmachung des Hinweises auf die Patenterteilung: **22.04.87 Patentblatt 87/17**

(84) Benannte Vertragsstaaten: **CH DE FR GB IT LI NL**

(56) Entgegenhaltungen:
**HOUBEN-WEYL: "Methoden der Organischen Chemie", Band 8, 1952, Seiten 563-566, Georg Thieme Verlag, Stuttgart, DE; "Sauerstoffverbindungen III"**

(73) Patentinhaber: **BAYER AG, Konzernverwaltung RP Patentabteilung, D-5090 Leverkusen 1 Bayerwerk (DE)**

(72) Erfinder: **Beitzke, Bernhard, Dr., Bergmannsweg 5, D-5060 Bergisch Gladbach 1 (DE)**

EP 0 165 521 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung eines Gemisches aus einem gegebenenfalls substituierten Zimtsäureester und einem gegebenenfalls substituierten β-Alkoxy-β-phenyl-propionsäureester durch Umsetzung eines gegebenenfalls substituierten Benzaldehyds mit einem gegebenenfalls substituierten Essigsäureester und mit einem Alkoholat in Gegenwart eines Alkohols. Das Estergemisch kann sauer oder alkalisch zu gegebenenfalls substituierter Zimtsäure verseift werden. Die Erfindung betrifft daher ebenfalls ein Verfahren zur Herstellung von gegebenenfalls substituierter Zimtsäure, bei dem zunächst das genannte Estergemisch hergestellt und dann verseift wird.

Die Kondensation von aromatischen Aldehyden mit Essigsäureestern (Claisen-Schmidt-Reaktion) ist im Prinzip bekannt (Ber. *23*, 976 (1890)). Diese Reaktion wird in Gegenwart von metallischem Natrium durchgeführt, um brauchbare Ausbeuten an Zimtsäureester zu erzielen. Die Gegenwart von Alkoholen, beispielsweise von Ethanol, gibt unbefriedigende Resultate, wie in der genannten Literatur berichtet wird: Neben geringer Ausbeute an Zimtsäureester wird dabei hauptsächlich die Cannizzaro-Reaktion des Benzaldehyds zu Benzoesäureester und Benzylalkohol beobachtet. Auch beim Einsatz von alkoholfreiem Natriummethylat kann diese Reaktion offensichtlich nicht ganz vermieden werden.

In überarbeiteter Form findet sich die Kondensation von Benzaldehyd mit Essigsäureethylester in Org. Synth., Coll. Vol. 1, S. 252, John Wiley and Sons, New York, 2nd Ed. 1956. Auch hier wird die Kondensation in Gegenwart von metallischem Natrium durchgeführt, das in umständlicher Weise unter Xylol aufgeschmolzen, durch einen Rührer zerschlagen und durch Abkühlung in feinteiligen Partikeln erhalten wird. Anschliessend wird das Xylol abdekantiert und Ethylacetat zugesetzt. Diese überarbeitete Verfahrensweise regt den Zusatz von sehr geringen Mengen Alkohol an, der nur etwa 4 bis 5,5% des Natriums zum Alkoholat umsetzen kann. Grössere Alkoholmengen gelten als schädlich in Bezug auf die Ausbeute.

Das Arbeiten mit metallischem Natrium ist jedoch technisch aufwendig und erfordert besondere Sicherheitsvorkehrungen, weshalb es für die industrielle Anwendung nicht in Frage kommt. In EP 44 976 wird die Herstellung von p-Hexoxy-zimtsäure-methylester beschrieben, bei der Natriummethylat als Kondensationsmittel eingesetzt wird. Auch hier wird die Gegenwart eines Alkohols während der Kondensationsreaktion vermieden. Um dies zu gewährleisten, wird das zunächst als methanolische Lösung eingesetzte Natriummethylat mit Toluol versetzt, und anschliessend destilliert man das Methanol im Gemisch mit Toluol azeotrop ab.

Es wurde nun gefunden, dass man in Gegenwart von Alkohol ein Gemisch der eingangs genannten Ester herstellen kann, wobei hohe Ausbeuten erzielt werden, obwohl aus der Literatur bekannt war, dass die Gegenwart von Alkoholen bei der Herstellung von Zimtsäureester völlig unbefriedigende Ausbeuten ergibt.

Es wurde ein Verfahren zur Herstellung eines Gemisches aus einem gegebenenfalls substituierten Zimtsäureester und einem gegebenenfalls substituierten β-Alkoxy-β-phenyl-propionsäureester gefunden, das dadurch gekennzeichnet ist, dass man einen gegebenenfalls substituierten Benzaldehyd mit einem gegebenenfalls substituierten Essigsäureester im Überschuss und mit einem Alkoholat in Gegenwart eines Alkohols umsetzt.

Im Verfahren der EP 44 976 kann zwar das Natriummethylat in Form einer methanolischen Lösung in das Verfahren eingebracht werden; dafür muss man jedoch den grossen Nachteil in Kauf nehmen, dass zur Entfernung des Methanols ein azeotropes Gemisch von Methanol und Toluol anfällt, das Aufarbeitungskosten verursacht.

Es wird angenommen, dass die im erfindungsgemässen Verfahren ablaufenden chemischen Reaktionen durch die folgenden Formelgleichungen wiedergegeben werden können, wobei das Beispiel der Kondensation von Benzaldehyd mit Essigsäuremethylester und Natriummethylat dargestellt wird:

(1) $C_6H_5-CHO + 2\ CH_3-COOCH_3 + NaOCH_3 \rightarrow C_6H_5-CH=CHCOOCH_3 + CH_3COONa + 2\ CH_3OH$

(2) $C_6H_5-CHO + 2\ CH_3-COOCH_3 + NaOCH_3 \rightarrow C_6H_5-CH(OCH_3)-CH_2COOCH_3 + CH_3COONa + CH_3OH$

Diese Kondensationsreaktion unter Einsatz von alkoholischen Alkoholat-Lösungen macht das Verfahren technisch leicht durchführbar. Neben dem gegebenenfalls substituierten Zimtsäureester wird ein gegebenenfalls substituierter β-Alkoxy-β-phenyl-propionsäureester gebildet.

Als erfindungsgemäss einsetzbare gegebenenfalls substituierte Benzaldehyde seien ausser dem nicht substituierten Benzaldehyd solche genannt, die ein- oder mehrfach, bevorzugt zweifach, besonders bevorzugt einfach substituiert sind, beispielsweise solche der Formel

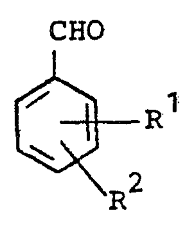 (I),

in der

$R^1$ und $R^2$ unabhängig voneinander für Wasserstoff, Alkyl, Cycloalkyl, Aryl, Aralkyl, Dialkylamino, Diarylamino, Halogen, Alkoxy, Aryloxy, Cycloalkoxy, Aralkoxy, Alkoxycarbonyl, Aryloxycarbonyl, Alkylcarbonyloxy, Alkylthio, Arylthio, Cycloalkylthio oder Aralkylthio stehen.

Alkylreste, auch in den sauerstoffhaltigen bzw. schwefelhaltigen Substituenten, sind beispielsweise solche mit 1-16, bevorzugt 1-10, besonders

bevorzugt 1-4 C-Atomen, wie Methyl, Ethyl, Propyl, Butyl, Hexyl, Decyl, Dodecyl, Hexadecyl.

Cycloalkylreste, auch in den sauerstoffhaltigen bzw. schwefelhaltigen Substituenten, sind solche mit 4-8, bevorzugt 5-6 im Ring angeordneten C-Atomen, die gegebenenfalls ein oder zwei Methyl- oder Ethylgruppen tragen können, wie Cyclobutyl, Cyclopentyl, Cyclohexyl, Cycloheptyl, Cyclooctyl, Methyl-cyclopentyl oder Methyl-cyclohexyl.

Als Arylreste, auch in den sauerstoffhaltigen bzw. schwefelhaltigen Substituenten, seien Phenyl, Naphthyl, Anthryl oder Diphenyl, bevorzugt Phenyl, zu verstehen.

Als Aralkylreste, auch in den sauerstoffhaltigen bzw. schwefelhaltigen Substituenten, seien solche aus einem Phenyl oder Naphthylkern und einer kurzen $C_1$-$C_4$-Alkylkette zu verstehen, wie Benzyl, Phenyl-ethyl oder Naphthyl-methyl, bevorzugt Benzyl.

Die Dialkylamino- bzw. Diarylamino-Substituenten tragen Alkyl- bzw. Arylgruppen der obengenannten Art.

Als Halogene seien beispielsweise Fluor, Chlor, Brom, bevorzugt Fluor und Chlor, genannt.

In bevorzugter Weise werden Benzaldehyde der Formel

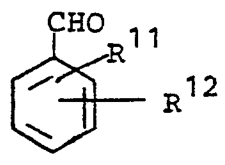

(II),

eingesetzt, in der

$R^{11}$ und $R^{12}$ unabhängig voneinander Wasserstoff, Alkyl, Cycloalkyl, Phenyl, Benzyl, Dialkylamino, Alkoxy, Phenoxy oder Benzyloxy bedeuten.

In besonders bevorzugter Weise werden Benzaldehyde der Formel

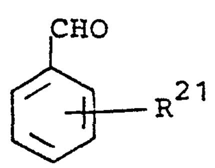

(III)

eingesetzt, in der

$R^{21}$ für Wasserstoff, Methyl, Ethyl, Dimethylamino, Methoxy, Ethoxy oder Phenoxy steht.

Als gegebenenfalls substituierte Essigsäureester für das erfindungsgemässe Verfahren seien beispielsweise solche der Formel

$$R^3CH_2-COOR^4 \qquad (IV)$$

genannt, in der

$R^3$ für Wasserstoff oder Alkyl steht und

$R^4$ Alkyl, Phenyl oder Benzyl bedeutet.

Als Alkyl sei geradkettiges oder verzweigtes Alkyl im Rahmen der obigen Offenbarung genannt. Die genannten Phenyl- bzw. Benzyl-Substituenten können durch Methyl, Ethyl, Methoxy, Ethoxy oder Dimethylamino substituiert sein.

In bevorzugter Weise werden Essigsäuremethylester, Essigsäureethylester, Essigsäurebutylester oder Essigsäure-iso-butylester eingesetzt.

Als Alkoholate für das erfindungsgemässe Verfahren seien beispielsweise solche der Formel

$$R^5OMe \qquad (V)$$

genannt, in der

$R^5$ geradkettiges oder verzweigtes Alkyl, Cycloalkyl oder Aralkyl bedeutet und

Me für ein Alkalikation oder ein Kationäquivalent der Metalle Magnesium, Aluminium oder Titan steht.

Die Bedeutung von Alkyl, Cycloalkyl und Aralkyl in der Formel (V) ist die gleiche wie die oben offenbarte. Die Alkylreste $R^5$ können ferner Alkoxygruppen oder Polyethergruppen enthalten, da auch Etheralkohole für die erfindungsgemäss einsetzbaren Alkoholate in Frage kommen.

Als Alkalimetalle seien beispielsweise Lithium, Natrium, Kalium, Rubidium und Cäsium, bevorzugt Lithium, Natrium oder Kalium, besonders bevorzugt Natrium genannt.

In bevorzugter Weise werden geradkettige oder verzweigte $C^1$-$C^4$-alkyl-Alkoholate von Natrium oder Kalium eingesetzt; in besonderer Weise seien Natriummethylat und Natriumethylat erwähnt.

Alkohole für das erfindungsgemässe Verfahren sind beispielsweise solche der Formel

$$R^6OH \qquad (VI),$$

in der

$R^6$ Alkyl, Cycloalkyl oder Benzyl bedeutet.

Der Bedeutungsumfang von Alkyl und Cycloalkyl ist der oben offenbarte. In bevorzugter Weise werden Methanol, Ethanol, Butanol und iso-Butanol eingesetzt.

Die Reste $R^4$, $R^5$ und $R^6$ sind grundsätzlich unabhängig voneinander. Es ist jedoch vorteilhaft, insbesondere wenn Umesterungen vermieden werden sollen, bei der Durchführung des Verfahrens den Resten $R^4$, $R^5$ und $R^6$ die gleiche Bedeutung zukommen zu lassen.

Für das erfindungsgemässe Verfahren verwendet man eine Lösung oder Suspension des Alkoholats in einem der genannten Alkohole, bevorzugt in dem Alkohol, von dem das Alkoholat abgeleitet ist, beispielsweise Natriummethylat in Methanol, Natriumethylat in Ethanol, Kalium-tert.-butylat in tert.-Butanol oder Natrium-isobutylat in iso-Butanol. Der Einsatz solcher Alkoholat-Lösungen macht das Verfahren technisch leicht durchführbar, denn solche Alkoholat-Lösungen sind leicht zugänglich und handhabbar und werden auch technisch hergestellt. Die Alkoholate werden im allgemeinen in Form einer 5-50 gew.-%igen, bevorzugt 20-40 gew.-%igen, besonders bevorzugt 25-35 gew.-%igen Lösung in dem zugrundeliegenden Alkohol eingesetzt. Handelsübliche technische Alkoholatlösungen sind im allgemeinen 30%ig, beispielsweise 30%iges Natriummethylat in Methanol. Alkalialkoholat-Lösungen der höheren Alkohole können durch Umalkoholisierung (Houben-Weyl, 4. Auflage, Band VI/2, Seite 13 (1963)) oder besonders vorteilhaft durch Entwässerung von Natriumhydroxid

und dem entsprechenden Alkohol hergestellt werden (EP 91 425).

Das Alkoholat wird in einer Menge von 1-2 Mol pro Mol des gegebenenfalls substituierten Benzaldehyds, bevorzugt in einer Menge von 1-1,2 Mol, eingesetzt. Der Alkohol wird in einer Menge von 2-25 Mol pro Mol Alkoholat, bevorzugt 3-15 Mol, eingesetzt. Der gegebenenfalls substituierte Essigsäureester wird im Reaktionsgemisch stets im molaren Überschuss über den gegebenenfalls substituierten Benzaldehyd gehalten, der bevorzugt mindestens 2 Mol Essigsäureester pro Mol Benzaldehyd beträgt, beispielsweise in einer Menge von 2-20 Mol pro Mol des gegebenenfalls substituierten Benzaldehyds, bevorzugt in einer Menge von 3-15 Mol, besonders bevorzugt 6-14 Mol, eingesetzt. Der überschüssige Essigsäureester kann nach beendeter Reaktion durch Destillation gemeinsam mit dem Alkohol zurückgewonnen und für einen neuen Ansatz verwendet werden.

Die Reaktion wird bei einer Temperatur von −20°C bis +150°C, bevorzugt bei 0-100°C, besonders bevorzugt bei 20-80°C durchgeführt. Das erfindungsgemässe Verfahren kann bei Normaldruck und/oder unter erhöhtem Druck ausgeführt werden. Vorteilhafterweise wird die Reaktion unter einem Schutzgas, beispielsweise unter Stickstoff durchgeführt, um Wasser (Luftfeuchtigkeit), Kohlendioxid und Sauerstoff auszuschliessen.

Die Reaktionsführung im erfindungsgemässen Verfahren kann in verschiedener Weise erfolgen. So kann in einem Rührgefäss die Alkoholatlösung vorgelegt werden und ein Gemisch aus dem gegebenenfalls substituierten Benzaldehyd und dem gegebenenfalls substituierten Essigester zudosiert werden. Man kann jedoch auch die Alkoholatlösung und den gegebenenfalls substituierten Essigsäureester vorlegen und den Benzaldehyd allein zudosieren. Weiterhin ist es möglich, den gegebenenfalls substituierten Essigsäureester vorzulegen und den Benzaldehyd und die Alkoholat-Lösung gleichzeitig, jedoch getrennt voneinander, zuzudosieren. Gute Ergebnisse werden weiterhin erhalten, wenn der gegebenenfalls substituierte Benzaldehyd und der gegebenenfalls substituierte Essigsäureester vorgelegt werden und die Alkoholatlösung zudosiert wird. Falls der gegebenenfalls substituierte Essigsäureester zudosiert wird, kann dieser zusätzlich noch Alkohol enthalten, der zusammen mit dem gegebenenfalls substituierten Essigsäureester aus einem früheren Ansatz zurückgeführt wird. Für den Fall, dass in einer der genannten Varianten das Alkoholat vorgelegt wird, kann dieses auch in Form einer Suspension vorgelegt werden und der Alkohol im Rahmen der obengenannten Menge gemeinsam mit einem der zuzudosierenden Reaktionspartner in das Reaktionsgemisch gebracht werden. In einer bevorzugten Ausführungsform wird die Alkoholatlösung vorgelegt und das Gemisch aus dem gegebenenfalls substituierten Benzaldehyd und dem gegebenenfalls substituierten Essigsäureester, gegebenenfalls zusammen mit dem Alkohol, zudosiert. In allen genannten Durchführungsvarianten wird darauf geachtet, dass der gegebenenfalls substituierte Essigsäureester stets im molaren Überschuss über dem gegebenenfalls substituierten Benzaldehyd vorliegt. Nach dem Eindosieren aller Reaktionspartner wird noch bis zur vollständigen Umsetzung nachgerührt. Das Ende der Umsetzung kann in bekannter Weise durch analytische Methoden, beispielsweise durch chromatographische Methoden, verfolgt werden.

Zur Aufarbeitung wird das Reaktionsgemisch im allgemeinen mit Wasser versetzt und angesäuert. Danach können die wässerige und die organische Phase getrennt werden, und die organische Phase kann beispielsweise durch Destillation zur Gewinnung des Estergemischs aus dem gegebenenfalls substituierten Zimtsäureester und dem gegebenenfalls substituierten β-Phenyl-β-alkoxy-propionsäureester aufgearbeitet werden. Vorteilhafterweise wird jedoch oft aus dem Reaktionsgemisch der überschüssige, gegebenenfalls substituierte Essigsäureester gemeinsam mit dem Alkohol soweit wie möglich abdestilliert, weil dieses Destillat in einem nächsten Reaktionsansatz ohne weitere Behandlung eingesetzt werden kann. Erst dann wird der verbleibende Reaktionsansatz mit Wasser in der angegebenen Weise versetzt und entweder durch Extraktion und Phasentrennung aufgearbeitet oder in einer weiter unten angegebenen Weise verseift. Das Estergemisch besteht im allgemeinen aus 80 bis 95% an gegebenenfalls substituiertem Zimtsäureester und aus 5 bis 20% an gegebenenfalls substituiertem β-Phenyl-β-alkoxy-propionsäureester.

Die gemeinsame Herstellung eines gegebenenfalls substituierten Zimtsäureesters und eines gegebenenfalls substituierten β-Alkoxy-β-phenyl-propionsäureesters ist neu. Die Herstellung eines β-Alkoxy-β-phenyl-propionsäureesters unter den Bedingungen der beschriebenen Kondensationsreaktion wurde bisher nicht für möglich gehalten.

Das Estergemisch findet, insbesondere wegen seines Gehaltes an dem gegebenenfalls substituierten Zimtsäureester, in der Parfüm- und Essenzenindustrie Verwendung (Ullmanns Encyclopädie der technischen Chemie, 4. Auflage, Band 24, Seite 592 (1983)). Selbstverständlich kann das Estergemisch, falls dies gewünscht wird, in seine Bestandteile, nämlich den gegebenenfalls substituierten Zimtsäureester und den gegebenenfalls substituierten β-Alkoxy-β-phenyl-propionsäureester, beispielsweise durch Destillation aufgetrennt werden.

Eine wichtige Weiterverarbeitung ist jedoch die Verseifung des gegebenenfalls substituierten Zimtsäureesters zur gegebenenfalls substituierten Zimtsäure. Hierbei erfolgt gleichzeitig die Eliminierung der β-Alkoxy-Gruppe aus dem gegebenenfalls substituierten β-Alkoxy-β-phenyl-propionsäureester. Diese Eliminierung erfolgt basisch oder sauer, bevorzugt mit wässerigen Basen.

Die Erfindung betrifft daher weiterhin ein Verfahren zur Herstellung von gegebenenfalls substituierter Zimtsäure, das dadurch gekennzeichnet ist, dass man zunächst in der angegebenen Weise einen gegebenenfalls substituierten Benzaldehyd mit einem gegebenenfalls substituierten Essigsäureester und mit einem Alkoholat in Gegenwart eines Alkohols zu dem angegebenen Gemisch der Ester umsetzt und dann das Estergemisch sauer oder alkalisch verseift.

Die Verseifung des Estergemischs erfolgt nach üblichen Methoden (Houben-Weyl, Band VIII, Seite 418). Die Verseifung wird bevorzugt alkalisch durchgeführt. Als Basen können beispielsweise wässerige Natronlauge oder wässerige Kalilauge, bevorzugt wässerige Kalilauge eingesetzt werden. Für die Verseifung kann sowohl das rohe Estergemisch aus dem gegebenenfalls substituierten Zimtsäureester und dem gegebenenfalls substituierten β-Alkoxy-β-phenyl-propionsäureester eingesetzt werden, das nach Abdestillieren des überschüssigen gegebenenfalls substituierten Essigsäureesters und des Alkohols erhalten wird, als auch das beispielsweise durch Destillation gereinigte Estergemisch. Die Base wird in einer Menge von 1-5 Mol, bevorzugt 1,5-2,5 Mol pro Mol der im Estergemisch vorhandenen Ester eingesetzt. Die Verseifung wird bei einer Temperatur von 20-150°C, bevorzugt 60-120°C, besonders bevorzugt bei 90-110°C durchgeführt. Aus dem Verseifungsansatz wird die freie Zimtsäure beispielsweise durch Ansäuern isoliert.

Zimtsäure und substituierte Zimtsäuren finden vielfältige Anwendung, beispielsweise zur Herstellung von Estern für die Parfüm- und Essenzenindustrie. Natriumcinnamat ist weiterhin ein Korrosionsschutzmittel (Ullmann, Band 24, Seite 592 (1983)). Zimtsäure ist weiterhin ein Zwischenprodukt für die Herstellung von Phenylalanin (JP 81/26197, zitiert nach Chem. Abstr. *95*, 95470b; JP 78/96388, zitiert nach Chem. Abstr. *89*, 213580p, JP 82/122834, Mitsubishi Petrochem.).

*Beispiel 1*

a) Herstellung des Estergemisches

In einem 2-l-Kolben legte man unter Stickstoff 210 g einer 30 gew.-%igen Natriummethylat-Lösung in Methanol vor. Dazu tropfte man in 70 min bei 60°C ein Gemisch von 111,4 g (1,05 mol) Benzaldehyd und 611 g (8,26 Mol) Methylacetat und rührte 6 Stunden bei 60°C. Dann versetzte man mit 210 ml 10%iger Essigsäure und trennte die Phasen. Die wässerige Phase wurde noch zweimal mit je 200 ml Toluol extrahiert, die organischen Phasen wurden vereinigt, über $Na_2SO_4$ getrocknet und destilliert. Nach dem Abdestillieren von Toluol und Methylacetat wurde bei 22 mbar und einer Kopftemperatur von ca. 128°C die Hauptfraktion abgenommen (132,8 g), die nach kombinierter Gaschromatographie/Massenspektroskopie-Analyse (GC/MS) folgende Zusammensetzung aufwies:

| | |
|---|---|
| Toluol | 1,1% |
| Benzaldehyd | 1,8% |
| Zimtsäuremethylester | 86,1% |
| Zimtsäure | 0,3% |
| β-Phenyl-β-methoxy-propionsäuremethylester | 10,7% |

Der Nachlauf (10,4 g) hatte folgende Zusammensetzung:

| | |
|---|---|
| Zimtsäuremethylester | 87,7% |
| Zimtsäure | 7,6% |
| β-Phenyl-β-methoxy-propionsäuremethylester | 4,7% |

Die Summe der Ausbeuten an Zimtsäuremethylester, Zimtsäure und β-Phenyl-β-methoxy-propionsäuremethylester betrug 80 Mol-%, bezogen auf Benzaldehyd-Einsatz.

Der β-Phenyl-β-methoxy-propionsäuremethylester hat folgendes $^1H$-NMR-Spektrum (250 MHz, $CDCl_3$, TMS int. Standard):

$\delta = 2{,}72$ (mc, 2H, $CH_2$), $\delta = 3{,}2$ (s, 3H, $OCH_3$), $\delta = 3{,}7$ (s, 3H, $COOCH_3$), $\delta = 4{,}7$ (mc, 1H, CH), $\delta = 7{,}3$-$7{,}65$ (m, 5H, ArH) ppm.

b) Verseifung

Die Hauptfraktion und der Nachlauf wurden vereinigt und mit 1120 g 10%iger Kalilauge (2 Mol) 6 Stunden unter Rückfluss erhitzt. Nach Ansäuern mit 48%iger Schwefelsäure und Trocknen wurden 122,8 g Zimtsäure, Schmelzpunkt 133°C, erhalten. Das entspricht einer Ausbeute von 79% der theoretischen Ausbeute.

*Beispiel 2*

In einem 2-l-Vierhalskolben mit Rührer, Thermometer, Tropftrichter und Rückflusskühler wurden unter Stickstoff 210 g einer 30%igen Natriummethylat-Lösung in Methanol vorgelegt. Bei 60°C wurde innerhalb von 1 Stunde eine Mischung aus 111 g (1,05 mol) Benzaldehyd und 593 g (8 mol) Methylacetat zugetropft. Man rührte 6 Stunden bei 60°C nach. Danach wurden 210 ml 10%ige Essigsäure zugegeben und die Phasen getrennt. Die wässerige Phase wurde noch zweimal mit je 200 ml Toluol extrahiert. Die organischen Phasen wurden vereinigt und mit 200 ml Wasser gewaschen. Nach Trocknen über $Na_2SO_4$ und Abdestillieren von überschüssigem Methylacetat und von Toluol wurden 199,6 g Rückstand erhalten, der nach GC folgende Zusamensetzung hatte:

| | |
|---|---|
| Toluol | 18,4% |
| β-Phenyl-β-methoxy-propionsäuremethylester | 12,5% |
| Zimtsäuremethylester | 69,1% |

Der Rückstand wurde mit 1120 g 10%iger Kalilauge versetzt und andestilliert. Nach Entfernen des Toluols wurde 6 h unter Rückfluss erhitzt. Nach Ansäuern mit 48%iger Schwefelsäure erhielt man 140 g Zimtsäure, Schmelzpunkt 133°C, Ausbeute 90% der theoretischen Ausbeute.

*Beispiel 3*

In einem 250 ml-Kolben wurden unter Stickstoff 21 g einer 30%igen Natriummethylat-Lösung in Methanol vorgelegt. Dazu tropfte man in 1 h bei 60°C Sumpftemperatur eine Mischung aus 11,1 g Benzaldehyd und 100 g (1,35 Mol) Methylacetat. Von der entstandenen Suspension wurde eine Probe genommen, in Methanol gelöst und durch Gaschromatographie/Massenspektrometrie untersucht. Neben Methylacetat, Spuren Benzaldehyd und dem Lösungsmittel enthielt die Probe 4,65% β-Phenyl-β-methoxy-propion-säuremethylester und 23,2% Zimtsäuremethylester.

*Beispiel 4*

In einem 2-l-Vierhalskolben mit Rührer, Thermometer, Tropftrichte und Rückflusskühler wurden unter Stickstoff 210 g einer 30%igen Natriummethylat-Lösung in Methanol vorgelegt. Bei 60°C wurde innerhalb von 1 h eine Mischung aus 111,4 g (1,05 mol) Benzaldehyd und 1000 g (13,51 mol) Essigsäuremethylester zugetropft. Anschliessend wurde noch 6 h bei 56°C gerührt. Dann wurde der überschüssige Essigester zusammen mit Methanol bis zu einer Kopftemperatur von 57°C abdestilliert (765,5 g). Man gab 500 ml Wasser zu und destillierte erneut bis zu einer Kopftemperatur von 91°C, wobei 220,7 g Destillat erhalten wurden.

Bei einer Sumpftemperatur von 90 bis 95°C wurden 612 g (2 mol) 18%ige Kalilauge zugetropft, worauf 6 h bei Rückfluss gerührt wurde. Nach Klärung mit 2,5 g Aktivkohle wurde mit 250 ml 48%iger Schwefelsäure bei 70°C auf pH 3 gestellt und die Zimtsäure ausgefällt. Man saugte bei 20°C ab und wusch dreimal mit je 200 ml Wasser nach. Nach dem Trocknen wurden 142 g Zimtsäure mit Schmelzpunkt 132-3°C erhalten, Ausbeute 91% der theoretischen Ausbeute.

*Beispiel 5*

In einem 2-l-Vierhalskolben mit Rührer, Thermometer, Rückflusskühler, Gabelstück und 2 Tropftrichtern wurden 1000 g Essigsäuremethylester unter Stickstoff vorgelegt und auf 55°C erwärmt. Dann wurden aus den Tropftrichtern getrennt, aber gleichzeitig 111,4 g (1,05 mol) Benzaldehyd und 210 g einer 30%igen Natriummethylat-Lösung in Methanol innerhalb 1 h zudosiert, wonach die erhaltene Suspension noch 6 h bei 55°C gerührt wurde. Nach Abdestillieren von 828,7 g Methylacetat/Methanol-Gemisch wurde mit 500 ml Wasser versetzt und weiter bis 92°C Kopftemperatur destilliert. Der Rückstand wurde mit 612 g (2 Mol) 18%iger Kalilauge versetzt und 6 h unter Rückfluss erhitzt. Nach Ausfällen und Isolieren der Zimtsäure, wie in Beispiel 4 beschrieben, wurden 133 g Zimtsäure erhalten, Schmelzpunkt 132-3°C, Ausbeute 85,5% der theoretischen Ausbeute.

*Beispiel 6*

Man verfuhr wie in Beispiel 4, setzte aber 296 g (4 mol) Essigsäuremethylester ein. Es wurden 125 g Zimtsäure, Schmelzpunkt 134°C erhalten; das entspricht einer Ausbeute von 80% der theoretischen Ausbeute.

*Beispiel 7*

Es wurden die gleichen Mengen wie in Beispiel 4 eingesetzt, nur wurde das Gemisch aus Benzaldehyd und Methylacetat vorgelegt und die Natriummethylat-Lösung in 1 h bei 60°C eindosiert. Man verfuhr weiter wie in Beispiel 4 und erhielt 126 g Zimtsäure mit Schmelzpunkt 132-134°C, Ausbeute 81% der theoretischen Ausbeute.

*Beispiel 8*

Man verfuhr wie in Beispiel 4, legte aber 360 g einer 30%igen Natriummethylat-Lösung vor. Zum Ausfällen der Zimtsäure wurden 340 ml 48%ige Schwefelsäure benötigt. Es wurden 141,8 g Zimtsäure, Schmelzpunkt 134°C, erhalten, Ausbeute 91% der theoretischen Ausbeute.

*Beispiel 9*

In einem 2-l-Vierhalskolben mit Rührer, Tropftrichter, Rückflusskühler und Thermometer wurden unter $N_2$ 200 g (1,1 mol) 30%ige Natriummethylat-Lösung in Methanol vorgelegt. Dazu wurde in 1 h bei 57-60°C Sumpftemp. eine Mischung aus 120,1 g (1 mol) p-Methylbenzaldehyd und 592 g (8 mol) Methylacetat getropft. Die entstandene Suspension wurde noch 6 h bei 58°C gerührt. Dann wurde das überschüssige Methylacetat zusammen mit dem Methanol abdestilliert (509 g).

Der Rückstand wurde mit 700 ml $H_2O$ versetzt und mit 100 ml 10%iger Essigsäure auf pH 6 gestellt. In dem erhaltenen zweiphasigen Gemisch kristallisierte die obere Phase langsam durch. Nach Absaugen und Trocknen wurden 147 g Substanz vom Schmp. 53-56° erhalten.

Analyse (GC):

92% p-Methylzimtsäuremethylester
3% p-Methylzimtsäure
5% β-Methoxy-β(4-methylphenyl)-propionsäuremethylester

Durch Extraktion der wässerigen Phase mit Xylol wurden weitere 10,8 g Substanz erhalten, Schmp. 53-56°C.

Analyse (GC):

85% p-Methylzimtsäuremethylester
10% p-Methylzimtsäure
5% β-Methoxy-β(4-methylphenyl)-propionsäuremethylester

Die Gesamtausbeute beträgt 89% der theoretischen Ausbeute.

## Patentansprüche

1. Verfahren zur Herstellung eines Gemisches aus einem gegebenenfalls substituierten Zimtsäureester und einem gegebenenfalls substituierten β-Alkoxy-β-phenyl-propionsäureester, dadurch gekennzeichnet, dass man einen gegebenenfalls substituierten Benzaldehyd mit einem gegebenenfalls substituierten Essigsäureester im Überschuss und mit einem Alkoholat in Gegenwart eines Alkohols umsetzt.

2. Verfahren nach Anspruch 1, dadurch gekennzeichnet, dass man 2-25 Mol des Alkohols pro Mol des Alkoholats einsetzt.

3. Verfahren nach Anspruch 1 und 2, dadurch gekennzeichnet, dass man 1-2 Mol des Alkoholats

pro Mol des gegebenenfalls substituierten Benzaldehyds einsetzt.

4. Verfahren nach Anspruch 1 bis 3, dadurch gekennzeichnet, dass der gegebenenfalls substituierte Essigsäureester stets im molaren Überschuss über den gegebenenfalls substituierten Benzaldehyd im Reaktionsgemisch gehalten wird.

5. Verfahren nach Anspruch 4, dadurch gekennzeichnet, dass stets mindestens 2 Mol des gegebenenfalls substituierten Essigsäureesters pro Mol des gegebenenfalls substituierten Benzaldehyds im Reaktionsgemisch vorhanden sind.

6. Verfahren nach Anspruch 1 bis 5, dadurch gekennzeichnet, dass als gegebenenfalls substituierter Benzaldehyd ein solcher der Formel (I) eingesetzt wird.

7. Verfahren nach Anspruch 1 bis 6, dadurch gekennzeichnet, dass als gegebenenfalls substituierter Essigsäureester ein solcher der Formel (IV) eingesetzt wird.

8. Verfahren nach Anspruch 1 bis 7, dadurch gekennzeichnet, dass ein Alkoholat der Formel (V) eingesetzt wird.

9. Verfahren nach Anspruch 1 bis 8, dadurch gekennzeichnet, dass ein Alkoholat der Formel (VI) eingesetzt wird.

10. Verfahren zur Herstellung von gegebenenfalls substituierter Zimtsäure, dadurch gekennzeichnet, dass man zunächst einen gegebenenfalls substituierten Benzaldehyd mit einem gegebenenfalls substituierten Essigsäureester und mit einem Alkoholat in Gegenwart eines Alkohols nach Anspruch 1 bis 9 zu einem Gemisch von gegebenenfalls substituiertem Zimtsäureester und gegebenenfalls substituiertem β-Alkoxy-β-phenyl-propionsäureester umsetzt und dann das Estergemisch sauer oder alkalisch verseift.

## Claims

1. Process for the preparation of a mixture of an optionally substituted cinnamic acid ester and an optionally substituted β-alkoxy-β-phenyl-propionic acid ester, characterised in that an optionally substituted benzaldehyde is reacted with an excess of an optionally substituted acetic acid ester and with an alcoholate in the presence of an alcohol.

2. Process according to Claim 1, characterised in that 2-25 moles of the alcohol are employed per mole of the alcoholate.

3. Process according to Claim 1 and 2, characterised in that 1-2 moles of the alcoholate are employed per mole of the optionally substituted benzaldehyde.

4. Process according to Claim 1 to 3, characterised in that the optionally substituted acetic acid ester is always kept in the reaction mixture in a molar excess over the optionally substituted benzaldehyde.

5. Process according to Claim 4, characterised in that at least 2 moles of the optionally substituted acetic acid ester are always present in the reaction mixture per mole of the optionally substituted benzaldehyde.

6. Process according to Claim 1 to 5, characterised in that the optionally substituted benzaldehyde employed is an optionally substituted benzaldehyde of the formula (I).

7. Process according to Claim 1 to 6, characterised in that the optionally substituted acetic acid ester employed is an optionally substituted acetic acid ester of the formula (IV).

8. Process according to Claim 1 to 7, characterised in that an alcoholate of the formula (V) is employed.

9. Process according to Claim 1 to 8, characterised in that an alcohol of the formula (VI) is employed.

10. Process for the preparation of optionally substituted cinnamic acid, characterised in that an optionally substituted benzaldehyde is first reacted with an optionally substituted acetic acid ester and with an alcoholate in the presence of an alcohol according to Claim 1 to 9 to give a mixture of optionally substituted cinnamic acid ester and optionally substituted β-alkoxy-β-phenyl-propionic acid ester, and the ester mixture is then hydrolysed under acid or alkaline conditions.

## Revendications

1. Procédé de production d'un mélange d'un ester d'acide cinnamique éventuellement substitué et d'un ester d'acide β-alkoxy-β-phényl-propionique éventuellement substitué, caractérisé en ce qu'on fait réagir un benzaldéhyde éventuellement substitué avec un ester d'acide acétique éventuellement substitué en excès et avec un alcoolate en présence d'un alcool.

2. Procédé suivant la revendication 1, caractérisé en ce qu'on utilise 2 à 25 moles d'alcool par mole de l'alcoolate.

3. Procédé suivant les revendications 1 à 2, caractérisé en ce qu'on utilise 1 à 2 moles de l'alcoolate par mole du benzaldéhyde éventuellement substitué.

4. Procédé suivant les revendications 1 à 3, caractérisé en ce que l'ester d'acide acétique éventuellement substitué est toujours maintenu en excès molaire par rapport au benzaldéhyde éventuellement substitué dans le mélange réactionnel.

5. Procédé suivant la revendication 4, caractérisé en ce qu'il y a toujours au moins 2 moles de l'ester de l'acide acétique éventuellement substitué par mole du benzaldéhyde éventuellement substitué dans le mélange réactionnel.

6. Procédé suivant les revendications 1 à 5, caractérisé en ce qu'on utilise comme benzaldéhyde éventuellement substitué un benzaldéhyde de formule (I).

7. Procédé suivant les revendications 1 à 6, caractérisé en ce qu'on utilise comme ester d'acide acétique éventuellement substitué un ester de formule (IV).

8. Procédé suivant les revendications 1 à 7, caractérisé en ce qu'on utilise un alcoolate de formule (V).

9. Procédé suivant les revendications 1 à 8, caractérisé en ce qu'on utilise un alcool de formule (VI).

10. Procédé de production d'acide cinnamique éventuellement substitué, caractérisé en ce qu'on fait réagir tout d'abord un benzaldéhyde éventuellement substitué avec un ester d'acide acétique éventuellement substitué et avec un alcoolate en présence d'un alcool suivant les revendications 1 à 9 pour former un mélange d'ester d'acide cinnamique éventuellement substitué et d'ester d'acide β-alkoxy-β-phényl-propionique éventuellement substitué et on saponifie ensuite le mélange d'esters dans des conditions acides ou alcalines.